# EUROPEAN PATENT SPECIFICATION

(11) **EP 3 384 289 B1**
(45) Date of publication and mention of the grant of the patent: **01.06.2022**
(21) Application number: 16823045.6
(22) Date of filing: 01.12.2016
(51) Int. Cl.: G01N 33/561, G01N 33/68

(54) **METHOD OF ANALYSIS OF PROTEINS IN URINE**
VERFAHREN ZUR ANALYSE VON PROTEINEN IN URIN
PROCEDE D'ANALYSE DES PROTEINES DANS L'URINE

(30) Priority: 02.12.2015 PL 41503315
(43) Date of publication of application: 10.10.2018
(73) Proprietor: Instytut Biochemii I Biofizyki Polskiej Akademii Nauk, 02-106 Warszawa (PL); Warszawski Uniwersytet Medyczny, 02-091 Warszawa (PL)
(72) Inventor: GRZESIUK, Elzbieta, 02-795 Warszawa (PL); PIWOWARSKI, Jan, 02-626 Warszawa (PL); PILZYS, Tomasz, 01-517 Warszawa (PL); WISNIOWSKA, Iga, 20-515 Prawiedniki (PL); MARCINKOWSKI, Michal, 68-100 Zagan (PL); GARBICZ, Damian, 39-310 Radomysl Wielki (PL); LISIK, Wojciech, 05-200 Wolomin (PL); KOSIERADZKI, Maciej, 04-048 Warszawa (PL); PACZEK, Leszek, 03-603 Warszawa (PL); KRASOWSKI, Pawel, 17-312 Drohiczyn (PL)
(74) Representative: Grzelak, Anna
(86) International application number: PCT/IB2016/057240
(87) International publication number: WO 2017/093920

(56) References cited:
- EP-A1- 0 545 128
- WO-A2-2013/166030
- US-A1- 2015 219 667
- PARIKH CHIRAG R ET AL: "Urine Stability Studies for Novel Biomarkers of Acute Kidney Injury", AMERICAN JOURNAL OF KIDNEY DISEASES, vol. 63, no. 4, 6 November 2013 (2013-11-06), pages 567-572, XP028831483, ISSN: 0272-6386, DOI: 10.1053/J.AJKD.2013.09.013
- K. BRAMHAM ET AL: "The non-invasive biopsy--will urinary proteomics make the renal tissue biopsy redundant?", QJM, vol. 102, no. 8, 24 June 2009 (2009-06-24) , pages 523-538, XP055346534, GB ISSN: 1460-2725, DOI: 10.1093/qjmed/hcp071

## Description

### Technical field

The invention relates to a method of proteins analysis in urine, comprising preparation of a urine sample and detection of proteins IgG antibodies and complement component C3 in a whole urine sample collected from a patient after kidney transplantation procedure; the urine sample is stored in a glass container and SDS is added to the urine sample before analysis to a concentration of around 1% (v/v); the analysis is performed by ex vivo diagnosis of the immunological kidney transplant rejection wherein the protein detection is performed with a Western blot technique. The invention is used especially for evaluating a risk of kidney transplant rejection.

### Background art

### Urine as a material for diagnostic tests

Urine is almost an ideal source for detection of biomarkers for many diseases, including cancers. It is easily available from almost all patients, its acquisition is relatively simple and does not require invasive procedures (literature reference no. (1)). Therefore, the analysis of urine composition (proteome, metabolome, and even the presence of certain cells) is a subject of intensive studies in laboratories worldwide. Despite these advantages, there are also problems associated with urine analysis. These include, among others, large amount of salts and other compounds, which can interrupt the appropriate signal, low protein concentration (in case of proteome tests), overall variability of urine composition resulting from differences in lifestyle and nutrition between particular individuals.

Urine is commonly used as a material for testing in clinical diagnostic to evaluate general condition of a patient or in screening. According to the standards for urine samples handling defined by the European Confederation of Laboratory Medicine (ECLM), urine is centrifugated in a refrigerated centrifuge (4°C) for 5 minutes in 400xg (2). The obtained pellet is evaluated by microscope, and the supernatant is subjected to further chemical and biochemical analysis, which is used among others for:
- Diagnosis of diseases of urinary tract and kidneys;
- Diagnosis of metabolic diseases, e.g. different types of porphyria;
- Diagnosis of multiple myeloma - Bence-Jones protein.

In case of proteome analysis, random protein aggregation and degradation becomes a problem, whereby even a single protein may be present in urine in a number of forms of varying solubility. Therefore, for reliable proteomic analysis it is necessary to have a complete urine sample, where the subject of analysis are both the soluble fraction, normally tested so far, and the insoluble fraction, which comprises patient's cells, bacteria and macromolecular aggregates, to date routinely removed from the analysis by the use of centrifugation procedure (literature reference (3) (4) (5) (6)).

### Analysis of transplant rejection

The occurrence of an episode of acute rejection of a transplanted organ within the first six months after transplant increases the risk of organ loss up to 50%. The acute kidney transplant rejection correlates with formation of interstitial fibrosis and tubular atrophy, i.e. is conducive to emergence of chronic allograft nephropathy, which is the main cause of organ loss within the first year from transplant.

The acute kidney transplant rejection is defined as a sudden deterioration in organ function associated with certain pathological changes in the transplant. There are two main forms of histologic acute rejection:
1. Acute cellular rejection - characterised in organ infiltration by T lymphocytes and other inflammatory cells.
2. Acute humoral rejection - characterised in tissue damage, caused by the activity of antibodies circulating in blood and directed against antigens of the organ donor and the presence of immunological evidence of this process (e.g. C4d deposits in the organ).

The early humoral type rejection occurs generally within the first several months after the transplant, especially in highly immunized patients. The most common abnormality is an increase in creatinine level. The final diagnosis is done on the basis of a kidney biopsy, and the humoral damage marker is a diffuse accumulation of anti-C4d antibody label in an immunofluorescence assay. On the basis of the clinical image and imaging methods, this type of rejection is easy to overlook and hard to distinguish from necrosis of renal tubules (ATN). Additionally, the humoral type rejection may be complicated by cellular rejection, which further hinders diagnosis and effective treatment.

Although the acute rejection process occurs mainly in the early postoperative period, in some cases the acute rejection process occurs several months or even years after the organ transplant. There is no distinct borderline distinguishing between early and late episodes of acute rejection. Nowadays, most of the transplantation centres assume the borderline between early and late rejection to be after the third month from organ transplant. The chronic rejection is a part of a general process called chronic allograft injury CAI and is the second main cause of transplant loss, after patient death from cardiovascular causes. From the beginning of 1970s, the transplanted kidney biopsy remains the golden standard for diagnosis of organ dysfunction and also for evaluation of adequacy of immunosuppression. The material for histopathology testing is most often collected using the core needle biopsy method.

Biopsies "based on indications" are performed in case of unsatisfactory graft function which cannot be explained by non-invasive methods and in case of deviations in general urine analysis, mainly proteinuria.

Around 1/3 of histologically confirmed episodes of acute rejection proceed subclinically, i.e. with correct creatinine values and no other clinical signs.

Kidney transplant in a highly immunized recipient has a high risk of antibody-mediated rejection (AMR) and even if no AMR occurs, the survival of the transplant is markedly decreased in this group, which can be associated with the process of chronic organ antibody-mediated rejection. Therefore, there is still a need for more sensitive methods based on identification of deleterious antibodies allowing precise selection of an organ to be transplanted.

Monitoring the presence of antibodies after transplantation is still not a routine operation, but a field of intensive studies. Particularly significant is the *de novo* production of anti-HLA antibodies, and not only DSA-specific (Donor-Specific Antibodies).

The identification of antibodies is performed using the fluorimetric method. Polystyrene microbeads are coated with HLA antigens, which bind serum derived antibodies. Next, fluorescently labelled IgG immunoglobulins, which bind to serum derived antibodies, are added to the system. Then, the fluorescent signal is analysed by a computer program providing information about the content of the anti-HLA antibodies in the serum. Depending on the type of the test, the microbeads are coated with one antigen (single antigen assay) or six antigens (screening assays and PRA (Panel Reactive Antibody)), which reflects the test resolution.

Anti-HLA antibodies occur *de novo* in 25.7% of recipients, wherein, depending on the method (ELISA, cytometry, Luminex), their detection ranges from 15.9% (ELISA) to 25.7% (Luminex). The anti-HLA antibodies with DSA specificity, which are present among other specific molecules generated *de novo,* affect graft survival the strongest. However, DSA antibodies are detected only in 16% of the anti-HLA(+) recipients. The low detectability of DSA-specific antibodies may be caused by their adsorption in the graft tissue.

Unfortunately, biopsy is an invasive procedure for the patient. In order to collect the sample, qualified medical personnel is required, an adequate apparatus, equipment and facilities necessary to carry out the procedure. For this reason, it is not possible to monitor the patient's condition on an ongoing basis, which often results in detection of transplant rejection only when the changes are irreversible.

The fact that there are differences in solubility and amount of IgG antibody in urine in healthy individuals and patients after kidney transplant could be used for transplant rejection diagnosis. In healthy individuals, the IgG elements are dissolved in urine and show a tendency to aggregate and precipitate. In case of patients after a kidney transplant, there is an increase in antibodies concentration as well as their spontaneous precipitation out of the solution after the surgery. This precipitation process is, however, a random phenomenon, so the use of only the pellet or the supernatant may give false results.

US 2015219667 A1 describes that elevated levels of anti-apoptotic cell IgG is an important contributor to and predictor of late graft rejection. The association between elevated levels of anti-apoptotic cell IgG antibodies and late transplant rejection forms a basis for diagnosing and treating patients at high risk of late transplant rejection.

WO 2013166030 describes the methods of detecting complement activation including steps of detecting a level of iC3b wherein the detecting involves specific interaction between the iC3b and a non-cross-reactive antibody thereto.

The publication K. Bramham et al, "The non-invasive biopsy--will urinary proteomics make the renal tissue biopsy redundant?", QJM, vol. 102, No. 8, 24 June 2009 (2009-06-24) describes the possibility of urinary proteomics as a tool for replacing the invasive tissue biopsy for diagnosis of renal disease.

EP 0545128 A1 discloses a protein assay wherein a colorimetric test strip is dipped in a mixed uncentrifuged urine sample.

The publication Parikh Chirag R et al, "Urine Stability Studies for Novel Biomarkers of Acute Kidney Injury", American Journal of Kidney Diseases, Vol. 63, No. 4, 06.1. 2013 discloses analysis of kidney injury markers performed in uncentrifuged urine samples using ELISA assay. The inventors of the present invention, surprisingly found that it is possible to use the whole (not centrifugated) urine for protein analysis (in particular, for antibodies or complement components (complement system components)). The routine centrifugation step, which is considered as a necessary standard, was replaced by mixing the samples with the aim of establishing a homogeneous suspension, which is the subject of proteomic analysis. The inventors have developed the method of sample analysis according to the invention, which allows to monitor the changes in protein level in patient urine, and, in a preferred embodiment of the invention, to monitor the changes in IgG antibody concentration in urine of patients after transplantation, to determine their condition.

### Disclosure of invention

The present invention is defined by the appended claims. Embodiments and descriptions no longer falling under the scope of the appended claims are considered merely as examples suitable for understanding the invention.

The invention relates to a use of not centrifugated whole urine, which comprises in comparisor with the routinely analysed centrifuged urine a higher number of specific proteins, for the purposes of diagnostic analysis of proteins, IgG antibodies and complement component C3.

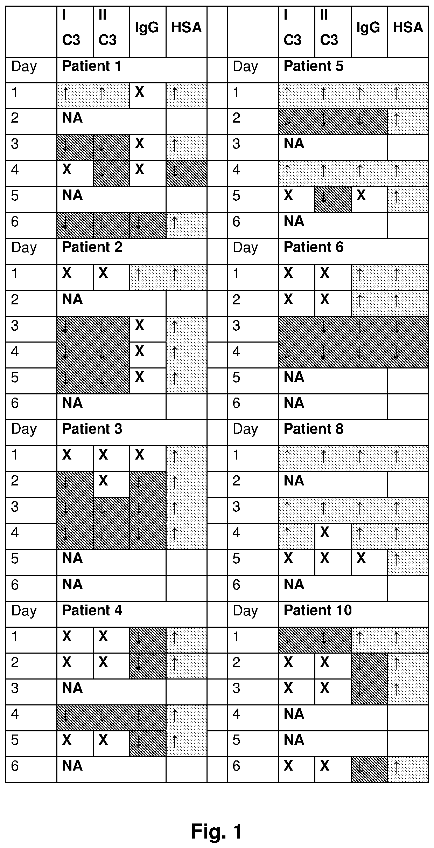

In the case of protein analysis, the problem is the tendency to a random protein aggregation and degradation, whereby even a single protein may be present in urine in a number of forms of varying solubility. As the inventors of the present invention have found, different protein behaviour in a centrifugated urine sample could be observed in different patients on different days (Fig. 1): transition into an insoluble aggregate phase (greater amount proteins after centrifugation are in the pellet - "↓"), or remaining in soluble phase (greater amount of proteins after centrifugation are in the supernatant - "↑") after centrifugation, which was demonstrated on the example of three proteins-HSA (Human Serum Albumin, literature reference no. (7)), C3 (complement component 3, literature reference no. (8)), IgG (Immunoglobulin G, literature reference no. (9)). According to the data shown on Fig. 1, aggregation and precipitation out of the solution, is in the case of proteins in urine a common phenomenon, which differ for each protein. HSA usually remains in soluble form, thus it is a marker of proteinuria, routinely tested in clinical diagnostics.

For the reasons presented above, for reliable proteomic analysis to a whole urine sample is essential, wherein the subject of an analysis are both the soluble fraction, which has been normally tested so far, and the insoluble fraction, which comprises patient's cells, bacteria and macromolecular aggregates, to date routinely removed from the analysis by the use of a centrifugation procedure.

The object of the invention is therefore a method of protein analysis in a urine sample, including preparation of the sample and protein detection, wherein the step of preparation of the urine sample for analysis includes mixing the sample without centrifugation, and protein detection is performed for whole urine, wherein the proteins are IgG antibodies and complement component C3 and analysis comprises monitoring changes in IgG antibody and complement component C3 levels, in whole urine collected from a patient after kidney transplantation procedure, wherein an increase in IgG antibody and complement component C3 levels, in time indicates a risk of transplant rejection, and decrease in IgG antibody and complement component C3 levels in time indicates a probable transplant acceptance;
and wherein the urine sample after collection is stored in a glass container and SDS is added to the urine sample before analysis to a concentration of around 1% (v/v);
and wherein the analysis is performed in an *ex vivo* diagnosis of the immunological kidney transplant rejection, wherein protein detection is performed with a Western blot technique.

The method according to the invention comprises following steps:
a) suspending the urine sample;
b) adding loading buffer to the urine sample;
c) protein aggregates breakdown, preferably through urine sample boiling or enzymatic cleavage;
d) applying the sample on a polyacrylamide gel and SDS-PAGE electrophoresis;
e) removal of excess salt from wells;
f) protein detection.

In the preferred method according to the invention the urine sample after collection and before analysis is frozen, and immediately before the analysis the sample is thawed in room temperature.

In the preferred method according to the invention step a) is performed by intense pipetting or using a Vortex.

In the preferred method according to the invention step c) is performed in temperature of 95°C for 2 minutes.

In the preferred method according to the invention step d) is performed in a 4-15% gradient polyacrylamide gel, and the electrophoresis runs in 1 x Tris-glycine- buffer for around 30 min at amperage of around 40 mA/gel.

The described method comprises therefore preparation of a urine sample for analysis, but without a centrifugation step and subsequent detection of analysed proteins.

In the described method, various known in the art methods for detection of analysed proteins can be used but this is not part of the present invention.

It is preferable to utilize a detection method, with a high enough specificity so that an additional interference source being cell-derived components does not pose a problem. Such a method is the Western blot method.

The method can be used in antibody analysis, for IgG antibodies. In another preferred embodiment, the protein is complement component C3.

The term "whole urine", as used herein, refers to urine comprising all elements found in the urinary bladder, among which there are also insoluble protein aggregates and epithelial and bacterial cells getting into the urine during excretion.

In an embodiment, the method can be used in an *ex vivo* diagnosis of the immunological kidney transplant rejection risk, which involves monitoring general IgG antibody levels and/or the C3 component levels in the whole urine samples, without a centrifugation step prior to protein detection.

Additionally, the inventors have shown that measurement done with the described method is stable independently of the sample storage method which ensures that the antibody level change in the case of patients' samples is induced by the organism immune reaction, and is not a preparation artifact.

The inventors of the invention have surprisingly found that the urine sample analysis with the method according to the invention enables a fast and reliable antibody level analysis in urine, in particular for early detection of the potential kidney transplant rejection. As mentioned above, the IgG antibody levels in urine increase in patients with rejection risk, whereas precipitation of these antibodies can occur randomly, therefore sample centrifugation and pellet analysis may give false results. This drawback is eliminated by the method according to the invention. Furthermore, the inventors have surprisingly found that there is a correlation between the changes in IgG antibody and C3 component levels in subsequent days after a kidney transplantation procedure, which suggests that IgG and C3 form a complex (Example 4).

In the preferred embodiment of the method, the analysis thus comprises monitoring of the changes in IgG antibody and complement component, in particular C3, levels, in whole urine collected from a patient after kidney transplantation procedure, wherein an increase in IgG antibody and complement, in particular C3, component levels time indicates a transplant rejection risk, and a decrease in IgG antibody and/or complement component C3, levels in time indicates a probable transplant acceptance. The method of protein analysis in a urine sample according to the invention allows to obtain a result in just a few hours.

The method according to the above mentioned embodiment of the invention has many advantages over transplant rejection risk analysis methods from the prior art.
- The method is non-invasive for patients;
- It enables sample collection from a patient even several times a day in order to monitor the transplant condition;
- It is relatively rapid, because the analysis results are obtained after a few hours;
- In comparison with previous methods, it is specific relative to immunological transplant rejection.

Additionally, the inventors have shown that the measurement carried out with the urine sample protein analysis method according to the invention is stable independently of the sample storage method, which ensures that the protein level change is not a preparation artifact, e.g. an IgG level change in the case of patients' samples is induced by an immunological reaction of the organism.

The protein analysis method in a urine sample involves the following steps:
a. suspending a urine sample;
b. adding a loading buffer to the urine sample which facilitates the application of protein samples on a gel;
c. protein aggregates breakdown, for example through urine sample boiling or enzymatic cleavage ;
d. applying the sample on a polyacrylamide gel and SDS-PAGE electrophoresis;
e. removal of excess salt from wells;
f. protein detection.

The person skilled in the art will readily choose a suitable loading buffer for step b). An example of the preferred loading buffer is the Laemmli buffer with the composition of: 2% SDS, 10% glycerol, 5% β-mercaptoethanol, 0.002% bromophenol blue, 0.125 M Tris-HCI, pH 6.8.

The urine sample is stored in a glass container after collection. SDS in the concentration of around 1% v/v is added to the urine sample before the analysis.

The urine sample after collection is frozen before the analysis, for example, in -20°C. if it needs to be stored longer than for around 16 hours. Before the analysis, the sample is preferably thawed in room temperature (~23°C).

Step a) may be performed, for example, by intense pipetting or using a Vortex.

Step c) is performed preferably at temperature of 95°C for 2 minutes.

Step d) is performed preferably in a 4-15% gradient polyacrylamide gel. The electrophoresis is preferably carried out in a 1x Tris-glycine buffer for around 30 min at amperage of around 40 mA/gel.

Step f) is performed by a Western blot technique. It is possible to use any other prior art suitable protein detection technique, such as, for example, mass spectrometry.

### Brief description of drawings

**Fig. 1** shows a comparison of protein behaviour in urine, transition to the insoluble aggregates phase (greater amount of proteins after centrifugation is in the pellet - "↓"), or remaining in the dissolved phase (greater amount of proteins after centrifugation is in the supernatant - "↑") after centrifugation, with the example of the three proteins - HSA, C3, IgG.

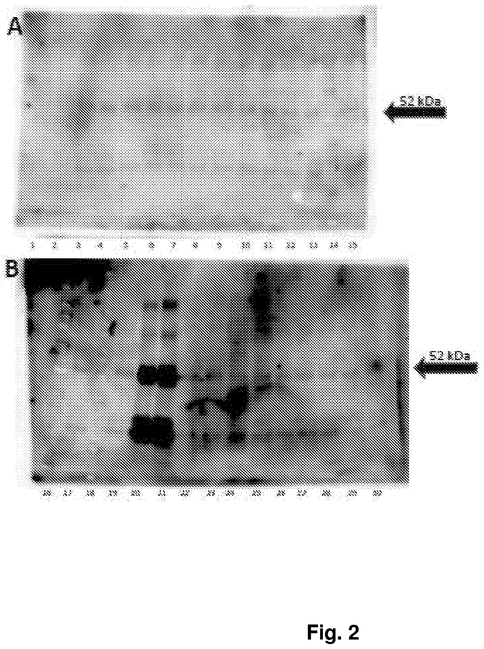

**Fig. 2** shows the results of urine samples analysis with a Western blot method after incubation in different conditions. A: 1- ION60/D, 2- ION60/G, 3- ION37/D, 4- ION37/G, 5- ION37/D, 6-ION37/G, 7- ION23/ZM, 8- ION23/ZM, 9- ION23/D, 10- ION23/G, 11- ION23/D, 12- ION23/G, 13- ION23/ZM, 14- M, 15- ION4/ZM; B: 1- M, 2- NI, 3- M, 4- NI, 5- TCA/ZAW, 6- TCA/ZAW, 7-W30/SUP, 8- W30/SUP, 9- W30/ZAW, 10- W30/ZAW, 11- ION-20/ZW, 12- ION4/G, 13-ION4/ZM, 14- ION4/G, 15- ION4/ZM. D - sample collected from the bottom part of the urine container; G - sample collected from the upper part of the urine container; ION-20 - overnight sample incubation at -20°C, ION4 - overnight sample incubation at 4°C; ION23 - overnight sample incubation at room temperature; ION37 - overnight sample incubation at 37°C; ION60 - overnight sample incubation at 60°C, M - molecular weight marker "PageRuler^{™} Unstained Protein Ladder" (the company ThermoFisher Scientific); ZM - sample collected from the middle part of the urine container after stirring with a vortex or by pipetting; NI - sample prepared without preincubation; SUP - supernatant after centrifugation; TCA - protein precipitation using the procedure employing trichloroacetic acid; W30 - sample centrifugation for 10 min at 30,000 g in 4°C; ZAW - suspending the solid phase in 160 µl of the Leamlie buffer.

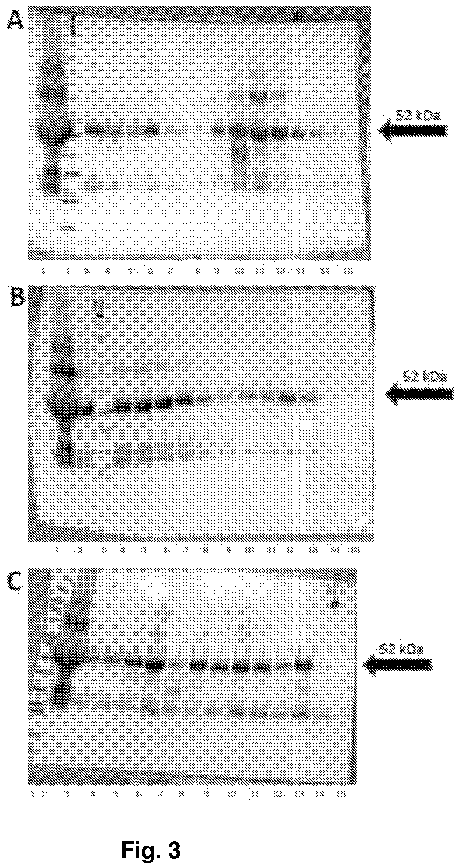

**Fig. 3** shows the changes in IgG antibody levels in urine from patients after kidney transplantation, based on the quantity of the IgG heavy chain analysis by the Western blot method. A: Contents of individual wells: 2: M; 3-8: Patient A 1, 2, 3, 6, 8, 9 days after the surgery; 9-15: Patient B 1, 2, 3, 4, 5, 7, 11 days after the surgery; B: Contents of individual wells: 3: M; 4-9: Patient C 2, 3, 4, 6, 9, 11 days after the surgery; 10-15: Patient D 1, 2, 3, 4, 7, 8 days after the surgery; C: Contents of individual wells: 1: M; 3-8: Patient E 1, 2, 3, 4, 7, 11 days after the surgery; 9-15: Patient F 1, 2, 3, 4, 6, 8, 11 days after the surgery.

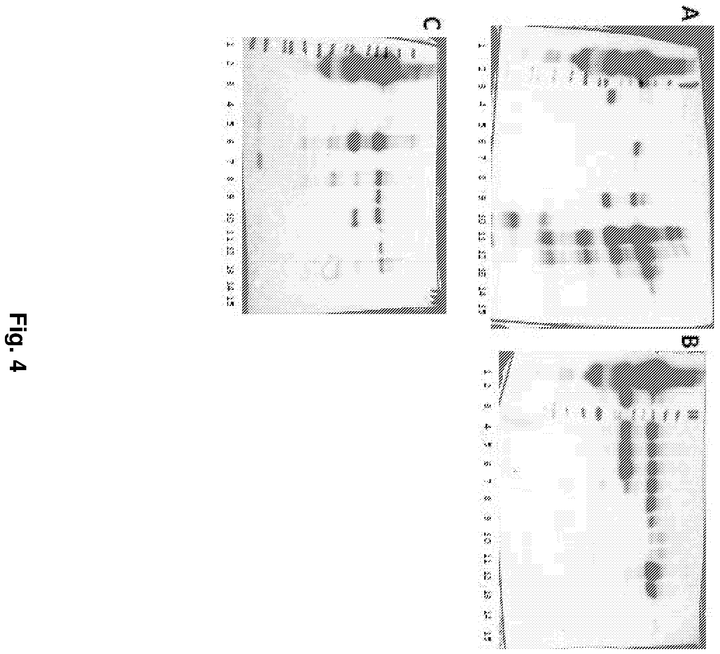

**Fig. 4** shows the changes in C3 component levels in urine from patients after kidney transplantation, based on the quantity of its two forms in the sample (75 kDa and 120 kDa) by the Western blot method. A: Contents of individual wells: 2: M; 3-8: Patient A 1, 2, 3, 6, 8, 9 days after the surgery; 9-15: Patient B 1, 2, 3, 4, 5, 7, 11 days after the surgery; B: Contents of individual wells: 3: M; 4-9: Patient C 2, 3, 4, 6, 9, 11 days after the surgery; 10-15: Patient D 1, 2, 3, 4, 7, 8 days after the surgery; C: Contents of individual wells: 1: M; 3-8: Patient E 1, 2, 3, 4, 7, 11 days after the surgery; 9-15: Patient F 1, 2, 3, 4, 6, 8, 11 days after the surgery.

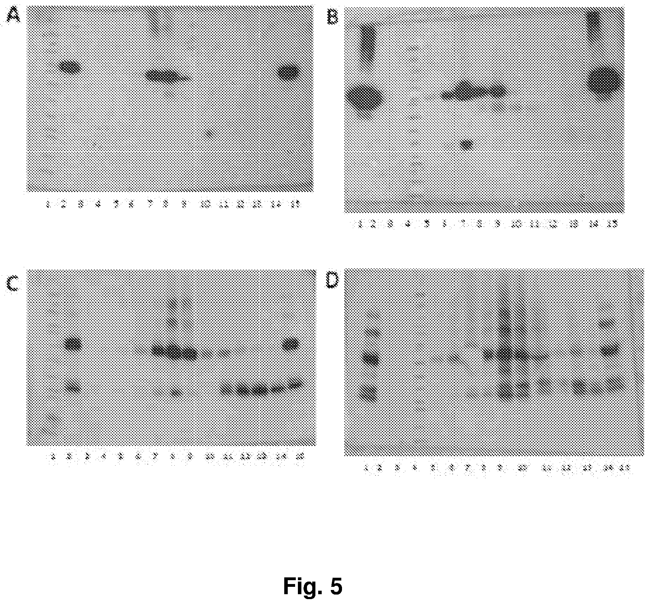

**Fig.** 5 shows fraction image after FPLC chromatography on a Size Exclusion column. The samples were analysed with the Western blot method for the presence of the various soluble forms in urine ,of a IgG antibody (blots C and D) and component C3 (blots A and B). Contents of the individual wells: A,C: 1: M; 2: Patient F urine supernatant sample (the second day after the surgery) applied on the column; 3-14: Fractions 1-12 from the column; 15: Patient's urine supernatant sample F (the second day after the surgery) applied on the column. B,D: 1: Patient C urine supernatant sample (the third day after the surgery) applied on the column; 2-3: Fractions 1-2 from the column; 4: M; 4-14: Fractions 3-14 from the column; 15: Patient C urine supernatant sample (the third day after the surgery) applied on the column.

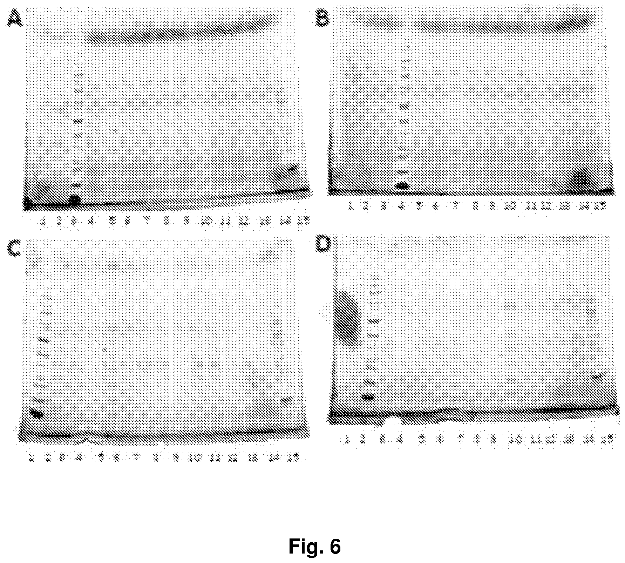

**Fig. 6** shows the effect of the container, in which the urine was stored, on the sample protein composition. The comparison of the whole urine incubated for an hour in a container with the elements attached to the container walls. The signal comes from the aromatic residues contained in the amino acids. The signal is recorded with the Bio-Rad ChemiDoc^{™} MP camera. A: Contents of individual wells: 1-2: Samples from the walls of the container, wherein initially the urine was collected; 3: M; 4: 1TK; 5: 1TR; 6: 1TS; 7: 1TN; 8: 1TSp; 9: 2TK; 10: 2TR; 11: 2TS; 12: 2TN; 13: 2TSp; 14: 3TK; 15: Patient C, urine from the 4th day after surgery; B: Contents of individual wells: 1: 3TR; 2: 3TS; 3: 3TN; 4: M; 5: 3TSp; 6: 4TK; 7:4TR; 8: 4TS; 9: 4TN; 10: 4TNSp; 11: 5TK; 12: 5TR; 13: 5TS; 14: 5TN; 15: 5TSp; C: Contents of individual wells: 1: M; 2: 1IK; 3: 1IR; 4: 1IS; 5: 1IN; 6: 1ISp; 7: 2IK; 8: 2IR; 9: 2IS; 10: 2IN; 11: 2ISp; 12: 5IK; 13: 5IR; 14: 5IS; 15: Patient C, urine from the 4th day after surgery; D: Contents of individual wells: 1: 3IK; 2: M; 3: 3IR; 4: 3IS; 5: 3IN; 6: 3ISp; 7: 4IK; 8: 4IR; 9: 4IS; 10: 4IN; 11: 4ISp; 12: 5IN; 13: 5ISp; 14: Sample from the wall of the container, wherein initially the urine was collected; 15: Patient C, urine from the 4th day after the surgery; Designations 1 - Non-sterile urine container (plastic); 2 - Sterile urine container (plastic); 3 - Sterile urine container (glass); 4 - Eppendorf, 5 ml; 5 - low bind Eppendorf, 5 ml; T - whole urine sample; I - sample taken from the inner walls of the container using a cotton bud; K - control container; R - two-fold dilution of the sample; S - sample with 1% SDS solution; N - coating the inner surface of the container with arinse aid; Sp - coating the outer surface of the container with a layer of an anti-electrostatic preparation.

### EXAMPLES

### Example 1 Urine sample analysis for the presence of IgG antibodies in patients after kidney transplantation procedure

An exemplary urine sample analysis performed with the method according to the invention comprises:
a) preparation of a urine sample
   - collecting urine from a patient according to a standard procedure (literature reference no. (2))
   - freezing the sample in -20°C (in the case of sample storage longer than 16 hours (literature reference no. (1))
   - transport to the laboratory
   - thawing in room temperature (~23°C)
   - suspending the urine sample by intense pipetting or stirring using a Vortex
   - adding Laemmli buffer (loading buffer) to the urine sample, so that the sample contains:
      - 2% SDS
      - 10% glycerol
      - 5% β-mercaptoethanol
      - 0.002% bromophenol blue
      - 0.125 M Tris-HCI
      - pH 6.8
   - boiling the sample in temperature of 95°C for 2 min
   - applying 10 µl of the sample on a 4-15% gradient polyacrylamide gel
   - SDS-PAGE in 1× Tris-glycine buffer for 30 min at constant amperage of 40 mA/gel
   - around 2 min after the beginning of the electrophoresis, when the sample proteins are in the gel, stopping the procedure and removal of excessive salt present in the wells by washing out.
b) Analysis of the change in IgG antibody levels in a urine sample using a Western blot method:
   - semi-dry transfer from the polyacrylamide gel to a nitrocellulose membrane using a buffer (96 mM Tris, 78 mM glycine, 0.075% SDS (w/v), 40% Methanol (v/v)) for a transfer of 1 h at a voltage of 10V,
   - blocking the membrane in 10% (w/v) solution of skimmed milk reconstituted in PBST (PBS buffer, pH 7.4 containing 0.1% (v/v) TWEEN^{®} 20 (CAS # 9005-64-5)) for 1 h,
   - decantation of the milk solution,
   - incubation in 5% (w/v) milk solution in PBST for 2 h with anti-IgG antibodies in concentration of 1:5000,
   - decantation of unbound antibodies and milk using PBST buffer (5 times 5 min each),
   - removal of TWEEN using PBS buffer (3 times 5 min each),
   - developing using a chemiluminescence reagent and a CCD camera.

The results of analysis of the urine samples derived from patients after kidney transplantation show the level of antibodies from the IgG group for a number of days. Based on the analysis of the change in antibody levels (represented by the band with a mass of about 52 kDa - heavy chain of IgG antibodies) there is an increase in antibody levels after the transplantation, which can be explained by the recipient's body immune reaction. Antibody levels reach maximum around the third-fifth day, and then begin to decline, which clearly is related with the beginning of the therapy with immunosuppressants to prevent transplant rejection.

### Example 2 Urine sample analysis for the C3 component presence in patients after the kidney transplantation

An exemplary urine sample analysis performed with the method according to the invention comprises:
c) Preparation of a urine sample;
   - collecting the urine from a patient according to a standard procedure (literature reference no. (2))
   - freezing the sample in -20°C (in case of sample storage longer than 16 hours (literature reference no. (1))
   - transport to the laboratory
   - thawing in room temperature (~23°C)
   - suspending the urine sample by intense pipetting or stirring using a Vortex
   - adding Laemmli buffer (loading buffer) to the urine sample, so that the sample contains the following:
      - 2% SDS
      - 10% glycerol
      - 5% β-mercaptoethanol
      - 0.002% bromophenol blue
      - 0.125 M Tris-HCI
      - pH 6.8
   - boiling the sample in temperature of 95°C for 2 min
   - applying 10 µl of the sample on a 4-15% gradient polyacrylamide gel
   - SDS-PAGE in 1× Tris-glycine buffer for 30 min at constant amperage of 40 mA/gel
   - around 2 min after the beginning of the electrophoresis, when the sample proteins are in the gel, stopping the procedure and removal of excessive salt present in the wells by washing out.
d) analysis of the change in C3 component levels in a urine sample using a Western blot method;
   - semi-dry transfer from the polyacrylamide gel to nitrocellulose membrane using a buffer (96 mM Tris, 78 mM glycine, 0.075% SDS (w/v), 40% Methanol (v/v)) for a transfer of 1 h at a voltage of 10V
   - blocking the membrane in 10% (w/v) solution of skimmed milk reconstituted in PBST (PBS buffer, pH 7.4 containing 0.1% (v/v) TWEEN^{®} 20 (CAS # 9005-64-5)) for 1 h
   - overnight incubation in 5% (w/v) milk solution in PBST with anti-C3 antibodies in concentration of 1:1000
   - decantation of unbound antibodies and milk using PBST buffer (5 times 5 min each).
   - incubation in 5% (w/v) skimmed milk solution in PBST for 3 h with secondary antimouse antibodies in concentration of 1:5000
   - decantation of unbound antibodies and milk using PBST buffer (5 times 5 min each).
   - removal of TWEEN using PBS buffer (3 times 5 min each)
   - developing using a chemiluminescence reagent and a CCD camera.

### Example 3 Examination of the method effectiveness for a variety of sample storage conditions

In order to test how external factors can affect the assays, different variants of sample handling were tested through introduction of modifications in the basic procedure. Urine derived from a control group comprising five subjects was examined. The following factors were taken into account:
a) Sample storage temperature:
   1) -20°C,
   2) 4°C,
   3) 23°C,
   4) 37°C,
   5) 60°C.
b) Sample incubation time:
   1) without incubation,
   2) overnight incubation.
c) Sample handling method:
   1) examination of urine suspension,
   2) examination of urine after 5 minutes sedimentation: sampling from the bottom and from the liquid surface,
   3) examination of the supernatant and pellet after centrifugation for 5 min at 14000 x g,
   4) protein precipitation in whole suspension using trichloroacetic acid (TCA).

In all cases, the IgG level in urine remained at a similar level (Fig. 2) independent of the sample preparation method (excluding overnight incubation in 60°C). Measurement is stable independent of the sample storage method, which ensures that the change in IgG levels in the case of patients' samples is induced by the immunological reaction of the organism, and not a preparation artifact.

An exemplary results for an analysis of the overall IgG antibody levels in patients' urine performed according to the invention are shown on Figure 3.

### Example 4 Determination in which soluble forms proteins present in urine may occur

To explain what the IgG level increase is related to, it was tested whether the protein forms complexes with other proteins present in urine. For this purpose, a Size Exclusion column separating proteins based on their weight was used. Then the detection was performed using a Western blot technique, in ta manner analogous to the previous samples.

An exemplary urine sample analysis involved:
a) preparation of a urine sample;
   - collecting the urine from a patient according to a standard procedure (literature reference no. (2))
   - freezing the sample in -20°C (in case of sample storage longer than 16 hours (literature reference no. (1))
   - transport to the laboratory
   - thawing in room temperature (~23°C)
b) chromatographic separation on a column
   - urine sample centrifugation for 5 min at 30,000 x g
   - applying 1 ml of the sample on the column and collecting fractions with a volume of 1 ml
   - protein precipitation using TCA
   - suspending the pellet using Laemmli buffer (loading buffer) with the following composition:
      - 2% SDS
      - 10% glycerol
      - 5% β-mercaptoethanol
      - 0.002% bromophenol blue
      - 0.125 M Tris-HCI
      - pH 6.8
   - boiling the sample in ttemperature of 95°C for 2 min
   - applying 10 µl of the sample on a 4-15% gradient polyacrylamide gel
   - SDS-PAGE in 1× Tris-glycine buffer for 30 min at constant amperage of 40 mA/gel
   - around 2 min after the beginning of the electrophoresis, when the sample proteins are in the gel, stopping the procedure and removal of excessive salt present in the wells.
c) analysis of the change in IgG antibody levels in a urine sample using a Western blot method;
   - semi-dry transfer from the polyacrylamide gel to nitrocellulose membrane using a buffer (96 mM Tris, 78 mM glycine, 0.075% SDS (w/v), 40% Methanol (v/v)) for a transfer of 1 h at a voltage of 10V.
   - blocking the membrane in 10% (w/v) solution of skimmed milk reconstituted in PBST (PBS buffer, pH 7.4 containing 0.1% (v/v) TWEEN^{®} 20 (CAS # 9005-64-5)) overnight
   - milk decantation
   - incubation in 5% (w/v) milk solution in PBST for 2 h with anti-IgG antibodies in concentration of 1:5000
   - decantation of unbound antibodies and milk using PBST buffer (5 times 5 min each)
   - removal of TWEEN using PBS buffer (3 times 5 min each)
   - developing using a chemiluminescence reagent and a CCD camera.
d) analysis of the change in C3 component levels in a urine sample using a Western blot method;
   - semi-dry transfer from the polyacrylamide gel to a nitrocellulose membrane using a buffer (96 mM Tris, 78 mM glycine, 0.075% SDS (w/v), 40% Methanol (v/v)) for a transfer of 1 h at a voltage of 10V.
   - blocking the membrane in 10% (w/v) solution of skimmed milk reconstituted in PBST (PBS buffer, pH 7.4 containing 0.1% (v/v) TWEEN^{®} 20 (CAS # 9005-64-5)) for an hour
   - overnight incubation in 5% (w/v) milk solution in PBST with anti-C3 antibodies in concentration of 1:1000
   - decantation of unbound antibodies and milk using PBST buffer (5 times 5 min each)
   - incubation in 5% (w/v) skimmed milk solution in PBST for 3 h with secondary antimouse antibodies in concentration of 1:5000
   - decantation of unbound antibodies and milk using PBST buffer (5 times 5 min each).
   - removal of TWEEN using PBS buffer (3 times 5 min each)
   - developing using a chemiluminescence reagent and a CCD camera.

The analysis of the fractions from the column shows that C3 components and IgG antibodies are present in the same fractions. It means that both proteins can be present in a complex with the same mass. The C3 component is a protein being a complement system element, i.e. of a biochemical pathway responsible for immunological transplant rejection, among others. There is also a correlation between the changes in IgG antibody and C3 component levels in subsequent days after the surgery, which suggests that IgG and C3 form a complex. This, in turn, leads to the conclusion, that the decrease in IgG antibody levels in the final days and transplant acceptance are related to each other, whereby the used method gives correct results. As demonstrated on Figure 1, both aforementioned proteins tend to aggregate and precipitate from solution. Therefore, the use of whole urine is justified.

### Example 5 Determination of the effect of the container on the proteome present in urine

### a) Sample collection

- Preparation of the containers for urine collection. Coating the inner surface with a layer of rinse aid and the outer surface with an anti-electrostatic preparation-;
- Urine collection according to a standard procedure (3 male subjects aged 25-27 years).
- Mixing together equal amounts of urine.
- Preparation groups of 5 of the following containers:
   1. Non-sterile urine container (plastic)
   2. Sterile urine container (plastic)
   3. Sterile urine container (glass)
   4. Eppendorf tube, 5 ml
   5. Low bind Eppendorf tube, 5 ml
- Container designation with K, R, S, N, Sp and performing following procedures with them:
   1. K - control container
   2. R - adding 2 ml miliQ
   3. N- coating the container inner surface with rinse aid
   4. Sp - coating the container outer surface with an anti-electrostatic preparation
- Transfer of 4.5 ml of mixed urine to new containers
- Adding 500 µl 10% (v/v) SDS solution to containers designated as "S"
- Sample incubation in room temperature for 1 h, with gentle stirring from time to time
- Removal of liquid using a pipette
- Soaking a cotton bud in Laemmli buffer
- Collecting the proteins attached to the walls with a cotton bud (out of the 25 containers described above, and the first three containers, into which urine was collected initially)
- Transfer of the cotton bud to the eppendorf tube
- Separation of the solution from the cotton bud using centrifugation
- Collection of the sample with buffer to a new tube
- Boiling the sample in temperature of 95°C for 2 min
- Further procedure is analogous to the one presented in example 1.

The comparison of the whole urine sample proteome with the one collected from the container wall (Fig. 6) revealed a different protein pattern on the gel. This means that a part of the proteins form a deposit on the inner container surface. This effect is the smallest in the case of a glass container and with a presence of the 1% (v/v) SDS solution in the whole urine sample.

### Literature

1. Thongboonkerd V. Practical Points in Urinary Proteomics. Journal of Proteome Research. 2007, 6(10), 3881-3890.
2. Delanghe J., Speeckaert, M. Preanalytical requirements of urinalysis. Biochemia Medica. 2014, 24(1), 89-104.
3. Decramer S. de Peredo, A. G., Breuil, B., Mischak, H., Monsarrat, B., Bascands, J. -., & Schanstra, J. P. Urine in clinical proteomics. Molecular and Cellular Proteomics. 2008, 7(10), 1850-1862.
4. Thongboonkerd V. & Saetun, P. Bacterial overgrowth affects urinary proteome analysis: Recommendation for centrifugation, temperature, duration, and the use of preservatives during sample collection. Journal of Proteome Research. 2007, 6(11), 4173-4181.
5. Bakun M. Niemczyk, M., Domanski, D., Jazwiec, R., Perzanowska, A., Niemczyk, S., Dadlez, M. Urine proteome of autosomal dominant polycystic kidney disease patients. Clinical Proteomics. 2012, 9(1).
6. Rodriguez-del Valle M. Quakyi, I. A., Amuesi, J., Quaye, J. T., Nkrumah, F. K., & Taylor, D. W. Detection of antigens and antibodies in the urine of humans with plasmodium falciparum malaria. Journal of Clinical Microbiology. 1991, 29(6), 1236-1242.
7. [Online] http://www.uniprot.org/uniprot/P02768.
8. [Online] http://www.uniprot.org/uniprot/P01024.
9. [Online] http://emedicine.medscape.com/article/136897-overview.

## Claims

1. A method of protein analysis in a urine sample, including preparation of the urine sample and protein detection, **characterized in that** the step of preparation of the urine sample for analysis includes mixing the sample without centrifugation, and protein detection is performed for whole urine, wherein the proteins are IgG antibodies and complement component C3 and analysis comprises monitoring changes in IgG antibody and complement component C3 levels, in whole urine collected from a patient after kidney transplantation procedure, wherein an increase in IgG antibody and complement component C3 levels, in time indicates a risk of transplant rejection, and decrease in IgG antibody and complement component C3 levels in time indicates a probable transplant acceptance;
and wherein the urine sample after collection is stored in a glass container and SDS is added to the urine sample before analysis to a concentration of around 1% (v/v);
and wherein the analysis is performed in an *ex vivo* diagnosis of the immunological kidney transplant rejection, wherein protein detection is performed with a Western blot technique.

2. The method according to claim 1, **characterized in that** it comprises following steps:
a) suspending the urine sample;
b) adding loading buffer to the urine sample;
c) protein aggregates breakdown, preferably through urine sample boiling or enzymatic cleavage;
d) applying the sample on a polyacrylamide gel and SDS-PAGE electrophoresis;
e) removal of excess salt from wells;
f) protein detection.

3. The method according to any of claims 1-2, **characterized in that** the urine sample after collection and before analysis is frozen, and immediately before the analysis the sample is thawed in room temperature.

4. The method according to any of claims 1-3, **characterized in that** step a) is performed by intense pipetting or using a Vortex.

5. The method according to any of claims 1-4, **characterized in that** step c) is performed in temperature of 95°C for 2 minutes.

6. The method according to any one of claims 1-5, **characterized in that** step d) is performed in a 4-15% gradient polyacrylamide gel, and the electrophoresis runs in 1 x Tris-glycine- buffer for around 30 min at amperage of around 40 mA/gel.

## Patentansprüche

1. Verfahren zur Proteinanalyse in einer Urinprobe, das die Aufbereitung der Urinprobe und den Proteinnachweis einschließt, **dadurch gekennzeichnet, dass** der Schritt der Aufbereitung der Urinprobe für die Analyse das Mischen der Probe ohne Zentrifugation einschließt und der Proteinnachweis für den gesamten Urin durchgeführt wird, wobei die Proteine IgG-Antikörper und die Komplementkomponente C3 sind und die Analyse die Überwachung von Änderungen der IgG-Antikörper- und Komplementkomponentenniveaus C3 umfasst, in Vollurin, der von einem Patienten nach einem Nierentransplantationsverfahren gesammelt wurde, wobei ein Anstieg der IgG-Antikörper- und Komplementkomponentenkonzentration C3 mit der Zeit ein Risiko der Transplantatabstoßung anzeigt und eine Abnahme der IgG-Antikörper- und Komplementkomponentenkonzentration C3 mit der Zeit eine wahrscheinliche Transplantatannahme anzeigt;
und wobei die Urinprobe nach der Sammlung in einem Glasbehälter aufbewahrt wird und der Urinprobe vor der Analyse SDS in einer Konzentration von etwa 1 % (v/v) zugesetzt wird;
und wobei die Analyse in einer *Ex-vivo*-Diagnose der immunologischen Nierentransplantatabstoßung durchgeführt wird, wobei der Proteinnachweis mit einer Western-Blot-Technik durchgeführt wird.

2. DasVerfahren nach Anspruch 1, **dadurch gekennzeichnet, dass** es folgende Schritte umfasst:
a) Aussetzung der Urinprobe;
b) Zugabe von Ladepuffer zu der Urinprobe;
c) Abbau von Proteinaggregaten, vorzugsweise durch Abkochen der Urinprobe oder enzymatische Spaltung;
d) Auftragen der Probe auf ein Polyacrylamidgel und SDS-PAGE-Elektrophorese;
e) Entfernung von überschüssigem Salz aus Brunnen;
f) Proteinnachweis.

3. Das Verfahren nach einem der Ansprüche 1 bis 3, **dadurch gekennzeichnet, dass** die Urinprobe nach der Sammlung und vor der Analyse eingefroren wird und unmittelbar vor der Analyse bei Raumtemperatur aufgetaut wird.

4. Das Verfahren nach einem der Ansprüche 1 bis 3, **dadurch gekennzeichnet, dass** Schritt a) durch intensives Pipettieren oder unter Verwendung eines Vortex durchgeführt wird.

5. Das Verfahren nach einem der Ansprüche 1-4, **dadurch gekennzeichnet, dass** Schritt c) bei einer Temperatur von 95°C für 2 Minuten durchgeführt wird.

6. DasVerfahren nach einem der Ansprüche 1-5, **dadurch gekennzeichnet, dass** Schritt d) in einem 4-15%igen Polyacrylamid-Gel durchgeführt wird und die Elektrophorese in 1 x Tris-Glycin-Puffer- für etwa 30 min bei einer Stromstärke von etwa 40 mA/Gel läuft.

## Revendications

1. Une méthode d'analyse des protéines dans un échantillon d'urine, comprenant la préparation de l'échantillon d'urine et la détection des protéines, **caractérisée en ce que** l'étape de préparation de l'échantillon d'urine pour analyse comprend le mélange de l'échantillon sans centrifugation, et la détection des protéines est effectuée pour l'urine entière, dans laquelle les protéines sont des anticorps IgG et le composant C3 du complément et l'analyse comprend le contrôle des changements dans les niveaux d'anticorps IgG et de composant C3 du complément, dans l'urine entière recueillie chez un patient après une procédure de transplantation rénale, dans laquelle une augmentation des niveaux d'anticorps IgG et de composant C3 du complément dans le temps indique un risque de rejet de transplantation, et une diminution des niveaux d'anticorps IgG et de composant C3 du complément dans le temps indique une acceptation probable de transplantation ;
et dans laquelle l'échantillon d'urine après la collecte est stocké dans un récipient en verre et du SDS est ajouté à l'échantillon d'urine avant analyse à une concentration d'environ 1% (v/v) ;
et dans laquelle l'analyse est effectuée dans un diagnostic ex *vivo* du rejet immunologique de la transplantation rénale, dans laquelle la détection des protéines est effectuée par une technique de Western blot.

2. La méthode selon la revendication 1, **caractérisée en ce qu'**elle comprend les étapes suivantes:
a) suspension de l'échantillon d'urine ;
b) ajout d'un tampon de chargement à l'échantillon d'urine ;
c) décomposition des agrégats de protéines, de préférence par ébullition de l'échantillon d'urine ou par clivage enzymatique ;
d) application de l'échantillon sur un gel de polyacrylamide et électrophorèse SDS-PAGE ;
e) élimination de l'excès de sel dans les puits ;
f) détection des protéines.

3. La méthode selon l'une quelconque des revendications 1-2, **caractérisée en ce que** l'échantillon d'urine après la collecte et avant l'analyse est congelé, et immédiatement avant l'analyse, l'échantillon est décongelé à température ambiante.

4. La méthode selon l'une quelconque des revendications 1-3, **caractérisée en ce que** l'étape a) est réalisée par pipetage intense ou à l'aide d'un Vortex..

5. La méthode selon l'une quelconque des revendications 1-4, **caractérisée en ce que** l'étape c) est réalisée à une température de 95°C pendant 2 minutes.

6. La méthode selon l'une quelconque des revendications 1-5, **caractérisée en ce que** l'étape d) est réalisée dans un gel de polyacrylamide à gradient de 4-15%, et l'électrophorèse se déroule dans 1 x tampon Tris-glycine pendant environ 30 min à un ampérage d'environ 40 mA/gel.
